# EUROPEAN PATENT SPECIFICATION

(11) **EP 3 979 930 B1**
(45) Date of publication and mention of the grant of the patent: **21.08.2024**
(21) Application number: 20821927.9
(22) Date of filing: 02.06.2020
(51) Int. Cl.: A61B 17/34, A61B 17/06, A61M 39/10, A61M 39/12, A61M 25/06, A61B 5/06, A61M 25/01, A61M 29/00, A61B 34/20, A61B 5/00

(54) **MICROINTRODUCER SYSTEM**
MIKROEINFÜHRUNGSSYSTEM
SYSTÈME DE MICRO-INTRODUCTEUR

(30) Priority: 12.06.2019 US 201962860569 P
(43) Date of publication of application: 13.04.2022
(73) Proprietor: Bard Access Systems, Inc., Salt Lake City, UT 84116 (US)
(72) Inventor: THOMPSON, Chase, Bountiful, UT 84010 (US); MISENER, Anthony, Kent, Bountiful, UT 84010 (US); MESSERLY, Shayne, Kaysville, UT 84037 (US)
(74) Representative: Hoffmann Eitle
(86) International application number: PCT/US2020/035754
(87) International publication number: WO 2020/251806

(56) References cited:
- EP-A1- 2 842 590
- WO-A1-2018/104162
- WO-A2-2019/036456
- US-A- 5 755 702
- US-A- 5 882 294
- US-A- 6 159 198
- US-A1- 2005 205 446
- US-A1- 2009 318 867
- US-A1- 2016 228 200
- US-A1- 2017 290 563
- US-B1- 6 379 346

## Description

### Related Art

US 2016/0228200 A1 discloses an optical shape sensing system comprising a hub to provide a known trackable curvature of an insertable guidewire.

### SUMMARY

The invention is defined by claims 1 and 2. Further embodiments of the invention are defined by the dependent claims. Methods as such are not claimed.

Embodiments disclosed herein are directed to a microintroducer system including a microintroducer and an adapter. The microintroducer can include an introducer sheath coupled to a handle, the handle and introducer sheath defining a channel therethrough. The adapter can include a receiver tube having a predetermined angled portion, the receiver tube defining a pathway from a proximal opening to the channel of the microintroducer. The adapter can be integral with the microintroducer or separately attached. The predetermined angled portion can include a plurality of curved portions to form a helical pathway. The predetermined angled portion can form any angle with a longitudinal axis of the microintroducer system, for example, between 45° and 90°, or in one embodiment about 70 °. When separately attached, the adapter includes an attachment mechanism configured to couple to a proximal connector of the microintroducer. The connection member can include a be a spin nut including a break line to permit removal of the spin nut from an inserted medical device. The receiver tube of the adapter includes an opening or slit to permit removal of the receiver tube from an inserted medical device. The microintroducer can further include an advancement mechanism disposed in a wall of the receiver tube, the advancement mechanism configured to translate a medical device through the microintroducer system. The dvancement mechanism can be a scroll wheel, which can be disposed adjacent the predetermined angled portion of the receiver tube in some embodiments.

The microintroducer system can further include a shape sensing stylet system, which can include a shape sensing stylet and an external device in communication therewith. The predetermined angled portion provides a calibration point for the shape sensing stylet. In embodiments in which the microintroducer system includes an advancement mechanism, the advancement mechanism can provide a fiduciary point for the external device to start measuring one of a deflection pattern and an inserted length of the shape sensing stylet. A method of shape sensing a vasculature of a patient can include advancing the shape sensing stylet into the receiver tube and through the predetermined angled portion, and calibrating a first deflection of the shape sensing stylet against an angle formed by the predetermined angled portion. The external device can use the first deflection to measure a deflection pattern of the shape sensing stylet, and can use the deflection pattern to determine a path of the vasculature. The external device can use the deflection pattern to determine a shape of the vasculature of the patient in three-dimensional (3D) space.

In some embodiments, the adapter can be separately coupled to a proximal end of a microintroducer for receiving an elongate medical device. The adapter includes a funnel opening and defines a channel with an angled portion, the angled portion including a predetermined angle. The angled portion positions the funnel opening away from the skin surface providing a convenient target with which to introduce an elongate medical device. The adapter can further include a scroll wheel. The scroll wheel can be positioned at the angled portion and can engage a distal tip of the elongate medical device. The scroll wheel can be used to pull the elongate medical device into the adapter, and through the angled portion. Thus, preventing the elongate medical device from buckling or kinking. Further the scroll wheel provides a continuous pressure, deflection and advancement rate through the adapter.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a microintroducer system including a microintroducer and an integral adapter including an angled portion.
FIG. 1B shows a microintroducer system including a microintroducer and a separate adapter including an angled portion.
FIG. 2 shows the adapter of FIG. 1B coupled to the microintroducer.
FIG. 3A shows one embodiment of the adapter.
FIG. 3B shows another embodiment of the adapter.
FIG. 4A shows a microintroducer system including a shape sensing stylet system in a first position.
FIG. 4B shows the microintroducer system of FIG. 4A with the shape sensing stylet system in a second position.
FIG. 5A shows a microintroducer system including a scroll wheel in accordance with some embodiments.
FIG. 5B shows the scroll wheel in isolation with a stylet.

### DETAILED DESCRIPTION

Reference will now be made to figures wherein like structures will be provided with like reference designations. It is understood that the drawings are diagrammatic and schematic representations of exemplary embodiments of the present invention, and are neither limiting nor necessarily drawn to scale.

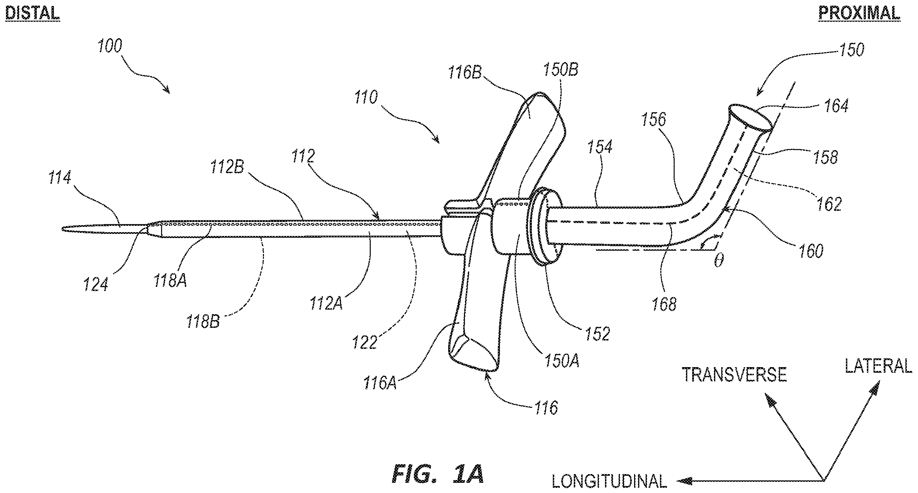

To assist in the description of the securement system, the following coordinate terms are used (see FIG. 1A). A "longitudinal axis" is generally parallel to the axis of an introducer sheath of the device. A "lateral axis" is normal to the longitudinal axis. A "transverse axis" extends normal to both the longitudinal and lateral axes. In addition, as used herein, "the longitudinal direction" refers to a direction substantially parallel to the longitudinal axis; "the lateral direction" refers to a direction substantially parallel to the lateral axis; and "the transverse direction" refers to a direction substantially parallel to the transverse axis. The term "axial" as used herein refers to the axis of the introducer sheath, and therefore is substantially synonymous with the term "longitudinal" as used herein.

For clarity it is to be understood that the word "proximal" refers to a direction relatively closer to a clinician using the device to be described herein, while the word "distal" refers to a direction relatively further from the clinician. For example, the tip of the introducer sheath placed within the body of a patient is considered a distal end of the device, while the connector remaining outside the body is towards a proximal end of the device. Also, the words "including," "has," and "having," as used herein, including the claims, shall have the same meaning as the word "comprising."

The terms "upper," "lower," "top," "bottom," "underside," "upperside" and the like, which also are used to describe the present securement system, are used in reference to the illustrated orientation of the embodiment. For example, the term "upperside" is used to describe the side of the device that is located above a lateral axis that passes through the axis of the introducer sheath. The term "underside" is used to describe the portion of the device that is located below a lateral axis that passes through the axis of the introducer sheath. The terms "left" and "right" are used consistently throughout the disclosure and are used to describe structures from the perspective of the clinician using the device.

Briefly summarized, embodiments disclosed herein are directed to microintroducer system and example methods thereof. Although the descriptions are in reference to a microintroducer for a vascular access system, it will be appreciated that embodiments described herein can also be applied to introducing gastric feeding tubes, catheters, guidewires, and similar systems of introducing devices and mapping internal areas of a human or animal body. Embodiments described herein include an adapter coupled to a proximal end of a microintroducer for receiving an elongate medical device, such as a shape sensing stylet. The adapter includes a funnel opening and defines a channel with an angled portion having a predetermined angle. The angled portion positions the funnel opening away from the skin surface providing a convenient target with which to introduce an elongate medical device. Further, the predetermined angle provides a fiduciary point for a medical device inserted through the microintroducer, such as a shape sensing stylet. As the stylet passes through the angled portion, an external device coupled to the shape sensing stylet calibrates the deflection of the stylet against the predetermined angle. Further the fiduciary point indicates a starting point with which to measure deflection patterns and inserted length of the shape sensing stylet. These can be used by the external device to determine a shape of the path of the shape sensing stylet and therefore a shape of the vasculature of the patient in three-dimensional (3D) space.

The adapter can further include a scroll wheel. The scroll wheel can be positioned at the angled portion and can engage a distal tip of the elongate medical device. The scroll wheel can be used to pull the elongate medical device into the adapter, and through the angled portion. Thus, preventing the elongate medical device from buckling or kinking. Further the scroll wheel provides a continuous pressure, deflection and advancement rate through the adapter. Embodiments herein further describe additional aspects of the system and example methods of use thereof.

FIG. 1A depicts a perspective view of a microintroducer system 100 including a microintroducer 110 and adapter 150 in accordance with an embodiment of the disclosure. The microintroducer 110 includes an introducer sheath 112 defining a channel 122 extending from a proximal end to a distal end, in which a dilator 114, or similar device can be disposed therein. In FIG. 1A, a distal tip of the dilator 114 is shown extending beyond a distal tip of the introducer sheath 112. A proximal end of the sheath 112 is shown coupled to a handle 116 (or pair of handles 116, e.g. left and right handle, 116A, 116B). The handle can be attached to the sides of the proximal end of the sheath, or the handle can form the proximal end of the channel. For example, the handle can be insert molded over the proximal end of the sheath. Moreover, the handle can include a valve. Examples of introducer sheaths, including the configurations of sheaths and handles, are disclosed in U.S. Patent No. 7,637,893, U.S. Patent No. 8,403,890, and U.S. Patent No. 8,932,260. The sheath 112 includes breach lines 118A, 118B extending longitudinally along apposing walls of the sheath 112. The breach lines 118A, 118B can be scorelines, perforations, laser cut lines, or similar lines of weakness that allow the sheath 112 to be splittable along a longitudinal axis. The breach lines 118A, 118B are disposed along opposing walls of the sheath 112 so that sheath 112 is splittable into two substantially equal halves. As shown in FIG. 1A, breach line 118A is disposed on an upper side of the sheath 112 with breach line 118B disposed on a lower side thereof, thus defining a sheath 112 splitable into a left and right portion, 112A, 112B. The left sheath portion 112A is coupled with a left handle 116A and the right sheath portion 112B is coupled with a right handle 116B. Alternatively, the sheath material (e.g., PTFE) can be formed (e.g., extruded) with an alignment of molecules so that the sheath can be peeled at the same or similar circumferential locations without the need for breach lines.

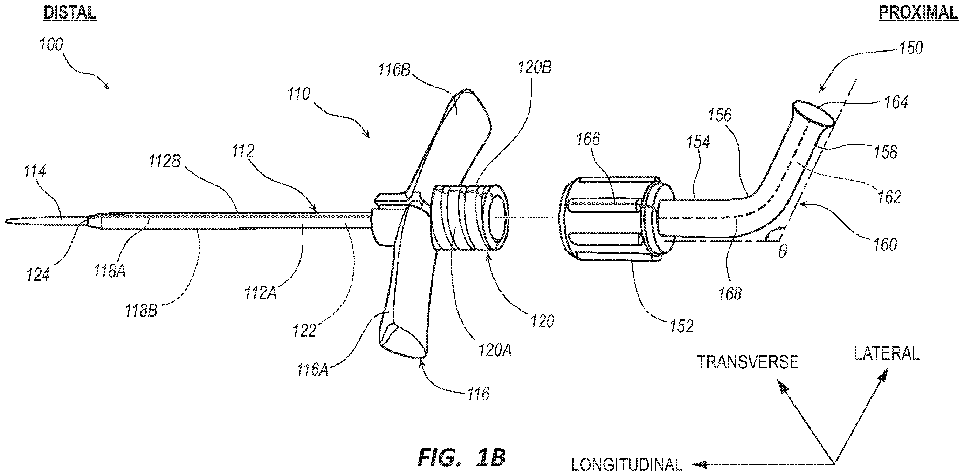

The adapter 150 can be integral or built into the microintroducer, as shown in FIG. 1A, or could be separately attached, as shown in FIG. 1B. When integral with the microintroducer 110, the adapter 150 includes a distal portion splittable into halves 150A and 15B that are aligned with the breach lines of the sheath 112 and/or the handle 116.

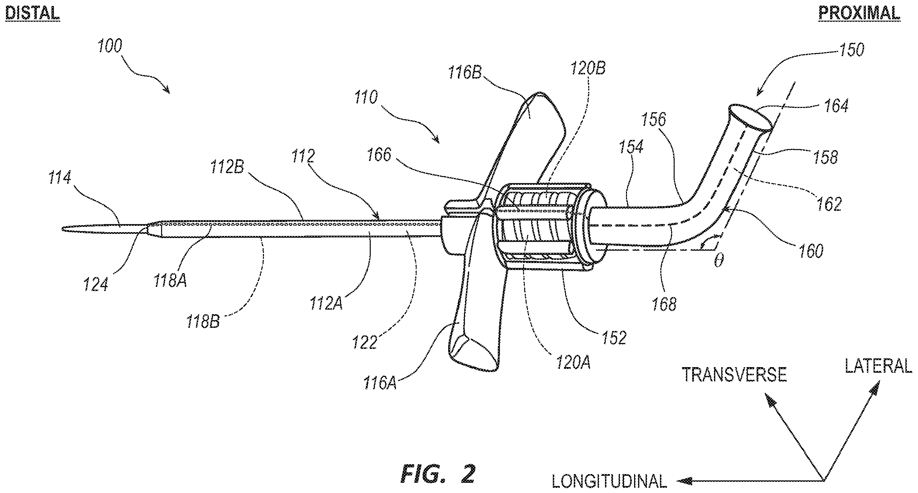

In embodiments in which the adapter 150 is separately attached, such as FIG. 1B, the microintroducer 110 further includes a connector 120 disposed at a proximal end. Connector 120 can include a threaded portion for receiving a corresponding spin nut 152 from adapter 150, as discussed below. However, it will be appreciated that other types of connectors are also contemplated, such as luer locks, slip fit, bayonet connectors, and the like. The connector 120 can be defined by a left and right portion 120A, 120B that are coupled with a proximal portion of the left and right handles 116A, 116B respectively. When assembled, as shown in FIG. 2, the left and right portions of the connector 120A, 120B define a continuous outer perimeter to allow a corresponding spin nut 152 or similar connector to engage an outer surface thereof.

As discussed, the adapter 150 can include an attachment mechanism to couple to the connector. The attachment mechanism in one embodiment includes a splittable or breakable portion to permit separation of the adapter 150 after a medical device is inserted therethrough and through the microintroducer into the vasculature of the patient. In one embodiment, shown in FIG. 1B, the adapter 150 includes a spin nut 152 configured for engaging threaded connector 120. In other embodiments, the attachment mechanism corresponds to different types of connectors as noted above. In the illustrated embodiment, the spin nut includes a break line 166 extending axially along a longitudinal axis. The break line 166 can include scorelines, perforations, laser cut lines, or similar lines of weakness, as described herein. In an embodiment, the spin nut 152 includes more than one break line 166, which allows the spin nut to be removed while maintaining an elongate medical device, extending therethrough, in place.

The adapter 150 includes a distal tube portion 154 that extends along a longitudinal axis of the microintroducer system 100, and in communication with the channel 122, to a predetermined formed angled portion 156 that smoothly curves at an angle to the distal tube portion 154 into a proximal tube portion 158. The proximal tube portion 158 extends away from the distal tube portion 154 at an angle to the longitudinal axis of the microintroducer system 100. The angled portion 156 can define an angle ("*Θ*") of between 5° and 175° from the longitudinal axis. In an embodiment, the angle *Θ* is between 45° and 90°, with a preferred embodiment being 70°. In some embodiments, the elongate tube includes a plurality of curved portions that together define a spiral path along the angled portion and, in some embodiments, also the proximal tube portion. Other curved pathways are also contemplated, such as a sinusoidal curve, for example. The distal tube portion 154, the angled portion 156, and the proximal tube portion 158 together define a continuous receiver tube 160 of the adapter 150. The receiver tube 160 defines a pathway 162 extending therethrough and is fluidly connected to the sheath channel 122 by way of the connector 120 in the illustrated embodiment. A proximal end of the receiver tube 160 includes a funnel 164. The funnel 164 defines a tapered portion such that a proximal end of the funnel 164 defines a larger diameter than a diameter of the receiver pathway 162.

The receiver tube 160 further includes a breach line 168. Breach line 168 extends axially along the receiver tube 160 from the funnel 164 at a proximal end to the spin nut 152 as a distal end. The breach line can include scorelines, perforations, laser cut lines, or similar lines of weakness as described herein, that allow the receiver tube 160 to be splittable along an axis of the receiver pathway 162. The breach line 168 allows the adapter 150 to be removed while allowing an elongate medical device, extending therethrough to remain in place. In an embodiment, the receiver tube 160 includes more than one breach line, for example 168A, 168B so that the adapter 150 can be separated into substantially equal portions. Although greater numbers of breach lines 168 defining greater numbers of portions of adapter 150 are also contemplated.

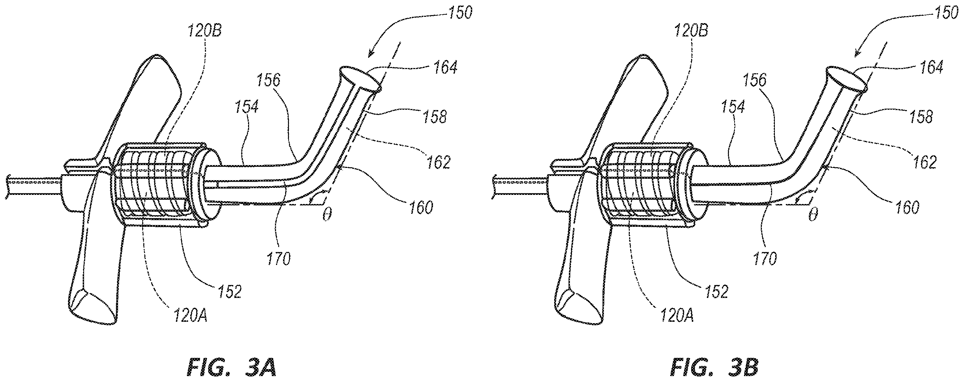

In an embodiment, as shown in FIG. 3A, the receiver tube 160 can include an elongate opening 170 in place of the breach line 168. The opening 170 in the embodiment shown in FIG. 3B is a slit such that opposing sides of the receiving tube 160 are in contact with one another. The opening 170 extends along lengthwise along at least one side of the receiver tube 160 along an entire length thereof, for example from the funnel 164 at a proximal end to the spin nut 152 as a distal end. The opening 170 allows the adapter to be removed while allowing any elongate medical device extending therethrough to remain in place. It will be appreciated that the opening 170 can be disposed at any circumferential location on the receiver tube. In the embodiment shown in FIG. 3A, the opening is shown as an equidistant gap between sides of the receiver tube 160. However, it should be appreciated that the gap can be tapered, and can be more narrow or wider than shown.

In an exemplary method of use, the microintroducer 110, with dilator 114 disposed therein, is used to access a vasculature, or similar area of a patient. The microintroducer 110 and dilator 114 are advanced distally until a distal tip 124 of the sheath 112 accesses the vasculature of the patient, or similar area. The dilator 114 is then removed and the sheath 112 of the introducer 110 can define an access path for other elongate medical devices to be introduced. Such elongate medical devices can include stylets, guidewires, catheters, such as integrated shape sensing catheters, peripheral IV catheter, midline catheter, Peripherally Inserted Central Catheter (PICC), acute or chronic Central Venous Catheter (CVC), and other elongate medical devices that are introduced into a patient.

In embodiments where the adapter is separate from the sheath/handle, i.e., not integral, the adapter 150 is then coupled with the connector 120 using spin nut 152 or similar connector. The receiver pathway 162 is fluidly connected with the sheath channel 122 to provide an access channel extending from funnel 164 at a proximal end to the sheath tip 124 at a distal end. The elongate medical device is then introduced to the vasculature by introducing the device through funnel 164, advancing along receiver pathway 162 / sheath channel 122 to extend beyond the distal tip 124 of sheath 112. With the elongate medical device in place, the microintroducer system 100 can be removed without disturbing the position of the elongate medical devices.

Initially, the adapter 150 is removed by unthreading the spin nut 152 from the connector, after which it is fractured along break line 166 and the receiver tube 160 is separated along breach line 168. In an embodiment, the spin nut 152 includes two break lines 166 disposed along opposite walls of the spin nut 152 so that the spin nut 152 fractures into two substantially equal portions, these can be separated and removed. Next, the user grasps the handles 116A, 116B and separates the handles in a proximal and transversely outward direction. This in turn causes the connector 120 and sheath 112 to separate along breach lines 118A, 118B (or along aligned molecules). This allows the microintroducer 110 to be removed leaving the elongate medical device in place.

In an embodiment, the receiver tube 160 is attached to the connector 120 using adhesive, bonding, welding, or similar suitable methods. In an embodiment, the receiver tube 160 and connector 120 are formed monolithically as a single structure. Accordingly, removal of the adapter 150 and the microintroducer 110 occurs concurrently. The receiver tube 160 and connector 120 will separate distally along breach line(s) 118 and proximally along breach line(s) 168 as the handles are pulled away from one another. This allows removal of the system 100 while leaving the elongate medical devices in place.

In embodiments having a separate adapter 150, as shown in FIG. 3A, the adapter 150 includes an elongate opening 170. With the spin nut 152 removed, as described herein, the adapter 150 is removed from the elongate medical device by allowing the elongate medical device to pass transversely, or laterally, through the opening 170, depending on the location of the opening 170. It will be appreciated that the opening may be disposed on an upper, lower, or side portion of the receiver tube 160 without departing from the scope of the present invention. The microintroducer 110 can then be removed by separating handles 116, as described herein.

Advantageously, the adapter 150, including funnel 164, provides a wider opening and a larger target area with which to introduce the elongate medical device. Further, when the microintroducer 110 is introduced to the patient, the microintroducer 110 substantially lies flat against a skin surface of the patient. The angled portion 156 allows the proximal tube portion 158 and funnel 164 to extend away from the skin surface providing a more convenient, open target area with which to introduce a medical device.

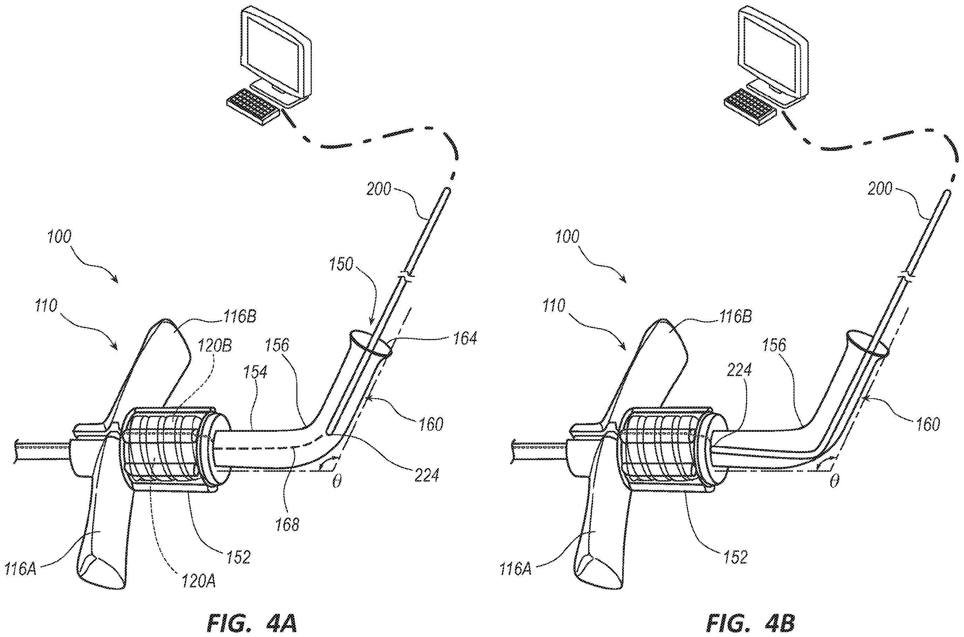

In an embodiment, the angle *Θ* provides a fixed reference angle that can be used as a fiduciary datum or initiation point for a fiber optic stylet 200. As shown in FIGS. 4A-4B, with the microintroducer system 100 disposed within a patient and the dilator 114 is removed, the microintroducer system 100 provides an access channel, that of receiver pathway 162 and sheath channel 122. The fiber optic stylet 200 can be advanced through the adapter until it is received within the angled portion 156. The angled portion 156 then deflects a distal tip 224 the fiber optic stylet 200 at a predetermined angle *Θ* to align a distal portion with a longitudinal axis of the microintroducer 110. The fiber optic stylet 200 is communicatively coupled with an external device 250 that detects and records any deflections along a length of the stylet 200. By passing the fiber optic stylet 200 through a known angle *Θ*, the external device 250 can automatically calibrate the deflection in the fiber optic stylet 200 against the known angle *Θ*. The fiber optic stylet 200 can also use the deflection of the known angle *Θ* as a start point in the process of shape sensing, such that any deflections detected along the stylet 200 that are distal to the deflection angle *Θ* can be recorded and used to map the path of the stylet 200 through the vasculature of the patient. The fiber optic stylet 200 can also use the deflection of the known angle *Θ* as a start point for tracking the length stylet that is inserted into the vasculature of the patient.

Fiber optic stylet systems, such as stylet 200 and external device 250, use the pattern of deflections along the length of the stylet, together with the length of stylet inserted, to determine a 3D map of the vasculature of the patient. Using the angled portion 156 of the adapter 150 as a fiduciary point allows the stylet 200 and external device 250 to calibrate the stylet to a reference plane and automatically initiate the mapping software. The external device 250 displays, on a user interface, an isometric view of the stylet 200 together with additional imagery of the patient.

As used herein the external device can include a handheld device, laptop, computer station, server, networked device or similar suitable device or devices communicatively coupled together, for receiving information from the stylet 200 and displaying a map of the path taken by the stylet through the vasculature of the patient. The external device 250 can further overlay imagery of the patient, the imagery can include CAT scans, PET scans, MRI, x-ray, or the like. As used herein, the fiber optic stylet 200 can be used in conjunction with one or more additional medical devices such as catheters or guidewires associated therewith. Accordingly, as the path of the stylet 200 is mapped, the path of any associated medical device can also be determined. It will also be appreciated that the fiber optic stylet system can include more than one stylet communicatively coupled with the external device 250 so as to map more than one path through the vasculature of the patient.

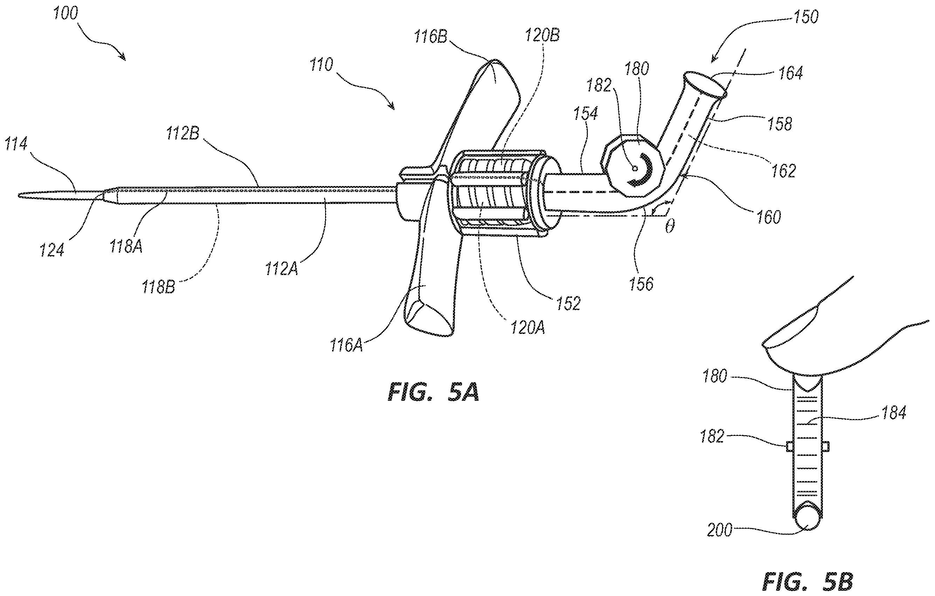

The adapter 150 can further include an advancement mechanism for the medical device to be inserted through the microintroducer 100. The advancement mechanism can be any type of driving mechanism, such as, for example, a slide, screw, crank, etc. In one embodiment, the advancement mechanism is a scroll wheel as shown in FIGS. 5A-5B. The scroll wheel 180 can be disposed within a wall of the adapter 150 so that an edge surface thereof engages an elongate medical device, e.g. stylet 200. Optionally, the stylet 200 can include a stiffening member (not shown). The stiffening member can include an elongate member extending longitudinally through or with the stylet 200, in an embodiment the stiffening member is co-extruded with the stylet 200. The stiffening member can include a material, such as a metal or similar alternative material, which displays stiffer mechanical properties that the stylet 200 and can further prevent the stylet 200 from buckling or kinking. The scroll wheel 180 rotates about a central point 182. As shown in FIG. 5B, a user manipulates an edge surface of the scroll wheel 180 that extends beyond an outer wall of the adapter 150 and is opposite that of the edge surface engaging the stylet 200. As the scroll wheel 180 is rotated, the stylet 200 is moved through the receiver pathway 162. As shown in FIG. 5A the scroll wheel 180 is position adjacent the angled portion 156 although other positions along the adapter 150 are also contemplated.

FIG. 5B shows a side profile of scroll wheel 180. In an embodiment the side surface defines a concave surface to engage a side surface of an elongate medical device, e.g. stylet 200. Optionally the scroll wheel 180 can include gripping features 184 such as ridges which can be formed monolithically with the scroll wheel 180, or be formed of a separate material displaying different characteristics. For example, gripping feature 184 can include a silicone rubber ring disposed about a side surface to aide engagement, not only of the stylet 200 but also for the user manipulating the opposite side. The scroll wheel 180 can further include a ball plunger and detent mechanism, or ratchet mechanism to provide a graduated rotation. This allows a user to rotate the scroll wheel 180 at set intervals and in turn advance or retract the stylet 200 at set distance intervals.

Advantageously, the scroll wheel 180 can allow a user to advance or retract the elongate medical device through the adapter 150 at a controlled rate. This prevents the stylet 200 from being advanced/retracted too quickly or at inconsistent rates. Further the scroll wheel 180 can prevent buckling of the medical device by pulling the elongate medical device through the adapter 150. As the elongate medical device is introduced to the adapter 150 a distal end engages the gripping features 184 of the scroll wheel 180 prior to engaging the angled portion 156. The scroll wheel 180 can maintain a tension on the stylet 200, or similar elongate medical device, and pull the device through the adapter 150 and about the known angle O of the angled portion 156. By contrast, when pushing the elongate medical device through the adapter 150 from a proximal end, a distal tip could potentially impinge on an inner wall of the angled portion 156 causing friction therebetween and buckling or kinking the device at a point proximal thereof.

The scroll wheel 180 can also apply a known, fixed strain on the elongate medical device. Where the stylet 200 detects deflections and strain forces applied along a length thereof, the known, fixed strain applied by the scroll wheel 180 can be accounted for and filtered from the results by the external device 250. This prevents the inconsistent strains and deflections of a user directly engaging the stylet 200 affecting the shape sensing results. The scroll wheel 180 can also provide a fiduciary point of known angle of deflection O for a calibration point and starting point for the shape sensing stylet 200, as described herein.

The described embodiments are to be considered in all respects only as illustrative, not restrictive. The scope of the invention is, therefore, indicated by the appended claims rather than by the foregoing description.

The invention is defined by the following claims.

## Claims

1. A microintroducer system (100), comprising:
a microintroducer (110) including an introducer sheath (112) coupled to a handle (116), the handle (116) and introducer sheath (112) defining a channel (122) therethrough; and
an adapter (150) comprising a receiver tube (160) having a predetermined angled portion (156), the receiver tube (160) defining a pathway (162) from a proximal opening to the channel (122) of the microintroducer (110),
wherein the adapter (150) includes a distal tube portion (154) that extends along a longitudinal axis of the microintroducer system (100), and in communication with the channel (122), to the predetermined angled portion (156) that smoothly curves at an angle to the distal tube portion (154) into a proximal tube portion (158),
wherein the adapter (150) is splittable along a breach line (168) extending along an entire length of the adapter (150).

2. A microintroducer system (100), comprising:
a microintroducer (110) including an introducer sheath (112) coupled to a handle (116), the handle (116) and introducer sheath (112) defining a channel (122) therethrough; and
an adapter (150) comprising a receiver tube (160) having a predetermined angled portion (156), the receiver tube (160) defining a pathway (162) from a proximal opening to the channel (122) of the microintroducer (110),
wherein the adapter (150) includes a distal tube portion (154) that extends along a longitudinal axis of the microintroducer system (100), and in communication with the channel (122), to the predetermined angled portion (156) that smoothly curves at an angle to the distal tube portion (154) into a proximal tube portion (158),
wherein the adapter (150) includes a longitudinal opening (170) extending along an entire length of the receiver tube (160).

3. The micro introducer system (100) according to claim 2, wherein the longitudinal opening (170) comprises a slit.

4. The microintroducer system (100) according to any of claims 1-3, wherein the adapter (150) is integral with the microintroducer (110).

5. The microintroducer system (100) according to any of claims 1-4, wherein the predetermined angled portion (156) includes a plurality of curved portions.

6. The microintroducer system (100) according to claim 5, wherein the pathway (162) is helical.

7. The microintroducer system (100) according to any of claims 1-6, wherein the predetermined angled portion (156) forms an angle with the longitudinal axis of the microintroducer system (100) between 45° and 90°, preferably wherein the angle is about 70°.

8. The microintroducer system (100) according to any of claims 1, 2 and 4-7, wherein the microintroducer (110) includes a proximal connector (120), and wherein the adapter (150) includes an attachment mechanism configured to couple to the proximal connector (120).

9. The microintroducer system (100) according to any of claims 1-8, wherein the adapter (150) includes an advancement mechanism disposed in a wall of the receiver tube (160), the advancement mechanism configured to translate a medical device through the microintroducer system (100).

10. The microintroducer system (100) according to claim 9, wherein the advancement mechanism is a scroll wheel (180), preferably wherein the scroll wheel (180) is disposed adjacent the predetermined angled portion (156).

11. The microintroducer system (100) according to any of claims 1-10, further comprising a shape sensing stylet system.

12. The microintroducer system (100) according to claim 11, wherein the shape sensing stylet system comprises a shape sensing stylet (200), and an external device (250) in communication with the shape sensing stylet (200).

13. The microintroducer system (100) according to claim 12, wherein the predetermined angled portion (156) provides a calibration point for the shape sensing stylet (200).

14. The microintroducer system (100) according to claim 12, wherein the adapter (150) includes an advancement mechanism, and wherein the advancement mechanism provides a fiduciary point for the external device (250) to start measuring one of a deflection pattern and an inserted length of the shape sensing stylet (200).

## Patentansprüche

1. Mikroeinführungssystem (100), umfassend:
eine Mikroeinführungsvorrichtung (110), die eine Einführungsschleuse (112) einschließt, die mit einem Griff (116) gekoppelt ist, wobei der Griff (116) und die Einführungsschleuse (112) einen Kanal (122) dort hindurch definieren; und
einen Adapter (150), der ein Aufnahmerohr (160) umfasst, das einen vorbestimmten abgewinkelten Abschnitt (156) aufweist, wobei das Aufnahmerohr (160) einen Pfad (162) von einer proximalen Öffnung zu dem Kanal (122) der Mikroeinführungsvorrichtung (110) definiert,
wobei der Adapter (150) einen distalen Rohrabschnitt (154) einschließt, der sich entlang einer Längsachse des Mikroeinführungssystems (100) erstreckt und mit dem Kanal (122) in Verbindung steht, bis zu dem vorbestimmten abgewinkelten Abschnitt (156), der sich gleichmäßig in einem Winkel zu dem distalen Rohrabschnitt (154) in einen proximalen Rohrabschnitt (158) krümmt, wobei der Adapter (150) entlang einer Bruchlinie (168), die sich entlang einer gesamten Länge des Adapters (150) erstreckt, teilbar ist.

2. Mikroeinführungssystem (100), umfassend:
eine Mikroeinführungsvorrichtung (110), die eine Einführungsschleuse (112) einschließt, die mit einem Griff (116) gekoppelt ist, wobei der Griff (116) und die Einführungsschleuse (112) einen Kanal (122) dort hindurch definieren; und
einen Adapter (150), der ein Aufnahmerohr (160) umfasst, das einen vorbestimmten abgewinkelten Abschnitt (156) aufweist, wobei das Aufnahmerohr (160) einen Pfad (162) von einer proximalen Öffnung zu dem Kanal (122) der Mikroeinführungsvorrichtung (110) definiert,
wobei der Adapter (150) einen distalen Rohrabschnitt (154) einschließt, der sich entlang einer Längsachse des Mikroeinführungssystems (100) erstreckt und mit dem Kanal (122) in Verbindung steht, bis zu dem vorbestimmten abgewinkelten Abschnitt (156), der sich gleichmäßig in einem Winkel zu dem distalen Rohrabschnitt (154) in einen proximalen Rohrabschnitt (158) krümmt,
wobei der Adapter (150) eine Längsöffnung (170) einschließt, die sich entlang einer gesamten Länge des Aufnahmerohrs (160) erstreckt.

3. Mikroeinführungssystem (100) nach Anspruch 2, wobei die Längsöffnung (170) einen Schlitz umfasst.

4. Mikroeinführungssystem (100) nach einem der Ansprüche 1-3, wobei der Adapter (150) mit der Mikroeinführungsvorrichtung (110) integral ist.

5. Mikroeinführungssystem (100) nach einem der Ansprüche 1-4, wobei der vorbestimmte abgewinkelte Abschnitt (156) eine Vielzahl von gekrümmten Abschnitten einschließt.

6. Mikroeinführungssystem (100) nach Anspruch 5, wobei der Pfad (162) spiralförmig ist.

7. Mikroeinführungssystem (100) nach einem der Ansprüche 1-6, wobei der vorbestimmte abgewinkelte Abschnitt (156) mit der Längsachse des Mikroeinführungssystems (100) einen Winkel zwischen 45° und 90° bildet, wobei der Winkel vorzugsweise etwa 70° beträgt.

8. Mikroeinführungssystem (100) nach einem der Ansprüche 1, 2 und 4-7, wobei das Mikroeinführungssystem (110) einen proximalen Verbinder (120) einschließt, und wobei der Adapter (150) einen Befestigungsmechanismus einschließt, der konfiguriert ist, um mit dem proximalen Verbinder (120) zu koppeln.

9. Mikroeinführungssystem (100) nach einem der Ansprüche 1-8, wobei der Adapter (150) einen Vorschubmechanismus einschließt, der in einer Wand des Aufnahmerohrs (160) angeordnet ist, wobei der Vorschubmechanismus konfiguriert ist, um eine medizinische Vorrichtung durch das Mikroeinführungssystem (100) zu bewegen.

10. Mikroeinführungssystem (100) nach Anspruch 9, wobei der Vorschubmechanismus ein Scrollrad (180) ist, wobei das Scrollrad (180) vorzugsweise benachbart zu dem vorbestimmten abgewinkelten Abschnitt (156) angeordnet ist.

11. Mikroeinführungssystem (100) nach einem der Ansprüche 1-10, weiter umfassend ein formabtastendes Mandrinsystem.

12. Mikroeinführungssystem (100) nach Anspruch 11, wobei das formabtastende Mandrinsystem einen formabtastenden Mandrin (200) und eine externe Vorrichtung (250), die mit dem formabtastenden Mandrin (200) in Verbindung steht, umfasst.

13. Mikroeinführungssystem (100) nach Anspruch 12, wobei der vorbestimmte abgewinkelte Abschnitt (156) einen Kalibrierungspunkt für den formabtastenden Mandrin (200) bereitstellt.

14. Mikroeinführungssystem (100) nach Anspruch 12, wobei der Adapter (150) einen Vorschubmechanismus einschließt, und wobei der Vorschubmechanismus einen Referenzpunkt für die externe Vorrichtung (250) bereitstellt, um mit der Messung eines von einem Ablenkungsmuster oder einer eingesetzten Länge des formabtastenden Mandrins (200) zu beginnen.

## Revendications

1. Système de micro-introducteur (100), comprenant :
un micro-introducteur (110) incluant une gaine d'introducteur (112) couplée à une poignée (116), la poignée (116) et la gaine d'introducteur (112) définissant un canal (122) à travers celui-ci ; et
un adaptateur (150) comprenant un tube de récepteur (160) présentant une partie inclinée prédéterminée (156), le tube de récepteur (160) définissant une voie (162) depuis une ouverture proximale vers le canal (122) du micro-introducteur (110),
dans lequel l'adaptateur (150) inclut une partie de tube distale (154) qui s'étend le long d'un axe longitudinal du système de micro-introducteur (100), et en communication avec le canal (122), vers la partie inclinée prédéterminée (156) qui s'incurve légèrement selon un angle vers la partie de tube distale (154) jusque dans une partie de tube proximale (158), dans lequel l'adaptateur (150) peut être divisé le long d'une ligne de rupture (168) s'étendant le long de toute la longueur de l'adaptateur (150).

2. Système de micro-introducteur (100), comprenant :
un micro-introducteur (110) incluant une gaine d'introducteur (112) couplée à une poignée (116), la poignée (116) et la gaine d'introducteur (112) définissant un canal (122) à travers celui-ci ; et
un adaptateur (150) comprenant un tube de récepteur (160) présentant une partie inclinée prédéterminée (156), le tube de récepteur (160) définissant une voie (162) depuis une ouverture proximale vers le canal (122) du micro-introducteur (110),
dans lequel l'adaptateur (150) inclut une partie de tube distale (154) qui s'étend le long d'un axe longitudinal du système de micro-introducteur (100), et en communication avec le canal (122), vers la partie inclinée prédéterminée (156) qui s'incurve légèrement selon un angle vers la partie de tube distale (154) jusque dans une partie de tube proximale (158),
dans lequel l'adaptateur (150) inclut une ouverture longitudinale (170) qui s'étend le long de toute la longueur du tube de récepteur (160).

3. Système de micro-introducteur (100) selon la revendication 2, dans lequel l'ouverture longitudinale (170) comprend une fente.

4. Système de micro-introducteur (100) selon l'une quelconque des revendications 1 à 3, dans lequel l'adaptateur (150) fait partie d'un seul tenant du micro-introducteur (110).

5. Système de micro-introducteur (100) selon l'une quelconque des revendications 1 à 4, dans lequel la partie inclinée prédéterminée (156) inclut une pluralité de parties incurvées.

6. Système de micro-introducteur (100) selon la revendication 5, dans lequel la voie (162) est hélicoïdale.

7. Système de micro-introducteur (100) selon l'une quelconque des revendications 1 à 6, dans lequel la partie inclinée prédéterminée (156) forme un angle avec l'axe longitudinal du système de micro-introducteur (100) entre 45° et 90°, de préférence dans lequel l'angle est d'environ 70°.

8. Système de micro-introducteur (100) selon l'une quelconque des revendications 1, 2 et 4 à 7, dans lequel le micro-introducteur (110) inclut un connecteur proximal (120), et dans lequel l'adaptateur (150) inclut un mécanisme de fixation configuré pour se coupler au connecteur proximal (120).

9. Système de micro-introducteur (100) selon l'une quelconque des revendications 1 à 8, dans lequel l'adaptateur (150) inclut un mécanisme d'avancement disposé dans une paroi du tube de récepteur (160), le mécanisme d'avancement étant configuré pour translater un dispositif médical à travers le système de micro-introducteur (100).

10. Système de micro-introducteur (100) selon la revendication 9, dans lequel le mécanisme d'avancement est une molette (180), de préférence dans lequel la molette (180) est disposée de manière adjacente à la partie inclinée prédéterminée (156).

11. Système de micro-introducteur (100) selon l'une quelconque des revendications 1 à 10, comprenant en outre un système de stylet de détection de forme.

12. Système de micro-introducteur (100) selon la revendication 11, dans lequel le système de stylet de détection de forme comprend un stylet de détection de forme (200) et un dispositif externe (250) en communication avec le stylet de détection de forme (200).

13. Système de micro-introducteur (100) selon la revendication 12, dans lequel la partie inclinée prédéterminée (156) fournit un point d'étalonnage pour le stylet de détection de forme (200).

14. Système de micro-introducteur (100) selon la revendication 12, dans lequel l'adaptateur (150) inclut un mécanisme d'avancement, et dans lequel le mécanisme d'avancement fournit un point de référence pour que le dispositif externe (250) démarre la mesure d'une caractéristique parmi une disposition de déviation et une longueur d'insertion du stylet de détection de forme (200).
